# EUROPÄISCHE PATENTSCHRIFT

(11) **EP 1 194 177 B1**
(45) Veröffentlichungstag und Bekanntmachung des Hinweises auf die Patenterteilung: **13.07.2005**
(21) Anmeldenummer: 00927000.0
(22) Anmeldetag: 19.04.2000
(51) Int. Cl.: A61M 1/10, F16J 15/43, F04D 3/02, F04D 13/06, F04D 29/08

(54) **Vorrichtung zur axialen Förderung von fluiden Medien**
Device for axially delivering fluidic media
Dispositif de refoulement axial de substances fluides

(30) Priorität: 20.04.1999 DE 19918840
(43) Veröffentlichungstag der Anmeldung: 10.04.2002
(73) Patentinhaber: Berlin Heart AG, 12247 Berlin (DE)
(72) Erfinder: NÜSSER, Peter, D-13051 Berlin (DE); MÜLLER, Johannes, D-10717 Berlin (DE); PETERS, Hans-Erhard, D-10437 Berlin (DE); BUSKE, Norbert, D-12437 Berlin (DE); NEUMANN, Werner, D-12247 Berlin (DE); GRAICHEN, Kurt, D-10115 Berlin (DE); KAUFFELDT, Conrad, D-12559 Berlin (DE)
(74) Vertreter: Golkowsky, Stefan
(86) Internationale Anmeldenummer: PCT/EP2000/003562
(87) Internationale Veröffentlichungsnummer: WO 2000/062842

(56) Entgegenhaltungen:
- WO-A-94/09835
- DE-A- 19 625 300
- US-A- 4 486 026
- US-A- 4 908 012
- US-A- 4 973 064
- US-A- 5 911 685
- YOSHINORI MITAMURA ET AL: "THE VALVO-PUMP AN AXIAL, NONPULSATILE BLOOD PUMP" , ASAIO TRANSACTIONS,US,HARPER AND ROW PUBLISHERS, HAGERSTOWN, MD, VOL. 37, NR. 3, PAGE(S) M510-M512 XP000298549 Seite 511, Spalte 1, Zeile 10 -Spalte 2, Zeile 10 Abbildung 2

## Beschreibung

Die Erfindung bezieht sich auf eine Vorrichtung zur axialen Forderung von fluiden Medien gemaß dem Oberbegriff des Anspruches 1.

Gattungsgemäße Vorrichtungen finden vorzugsweise Anwendung als Pumpe zur schonenden Förderung von Körperflüssigkeiten in der Medizin, bei chemischen, biologischen und/oder biochemischen Verfahren. Besondere Bedeutung haben diese Pumpen als Blutpumpen zur Unterstützung eines erkrankten Herzens, die in den Brustraum eines Patienten implantierbar sind.

In der Veröffentlichung Heart Replacement Artificial Heart 5, Seiten 245 - 252, Springer Verlag Tokyo 1996, Herausgeber T. Akutso und H. Koyagani, ist eine axial fördernde Blutpumpe zur Unterstützung eines erkrankten Herzens beschrieben worden. Diese Blutpumpe besitzt ein rotierendes Laufrad mit einer Beschaufelung, das innerhalb eines blutführenden rohres gelagert und mittels eines Elektromotors angetrieben wird. Hierzu ist das Laufrad als Rotor des Elektromotors ausgebildet und über an der Beschaufelung angebrachte Magnete mit dem außerhalb des Rohres angebrachten Stator des Elektromotors gekoppelt. Eine derartige Anordnung ist auch aus der US 4,957,504 bekannt. Vor und hinter dem Laufrad sind jeweils gehäusefest eine Leiteinrichtung mit Leitgitter angeordnet, die der Strömungsbeeinflussung dienen. Diese dort beschriebene Pumpe weist verschiedene Nachteile auf. Durch die räumliche Trennung im Motor, von Stator und Rotor des Elektromotors entstehen nicht unerhebliche Verluste bezüglich der Leistung des Elektromotors. Ein weiterer Nachteil entsteht durch die Anordnung des Stators des Elektromotors außerhalb des blutführenden Rohres. Die dadurch unvermeidliche Volumenvergrößerung der Gesamtvorrichtung kann die Implantierbarkeit beeinträchtigen. Weiterhin erfährt das geförderte Blut in nicht unerheblichem Ausmaße eine Traumatisierung und Schädigung. Das ist insbesondere zurückzuführen auf Scherungen und Verwirbelungen des Blutes, hervorgerufen durch Spalte zwischen dem äußeren Rand der Beschaufelung und der Innenseite des umgebenden blutführenden Rohres als auch durch die Anordnung von axialen Lagern.

Aus dem Artikel Yoshinori Mitamura et al, The Valvo Pump, An Axial Nonpulsatile Blood Pump, Asaio Transactions, Vol. 37 (1991), No. 3, S. 510 - 512, der zur Abgrenzung des Oberbegriffs des Anspruchs 1 herangezogen worden ist, ist eine Blutpumpe bekannt, die aus einer rohrförmigen Hohlkörperanordnung besteht, in deren Förderbereich ein in Rotation versetzbares Laufrad mit Beschaufelung und ein Motor mit Motorhalterung angeordnet sind. Die Motorhalterung ist als Fluid-Leiteinrichtung ausgebildet. Das Laufrad ist axial kraft- und formschlussig mit dem Motor verbunden. Die Welle ist an ihrem Lager mit einer Teflondichtung oder mit einer Magnetflussigkeitsdichtung abgedichtet, welche innerhalb des Motorgehauses angeordnet ist. Eine Scherung des empfindlichen Blutes zwischen Laufrad und Motor wird hierdurch nicht verhindert.

Die WO 94/09835 A1 zeigt ebenfalls eine Blutpumpe, welche jedoch ebenfalls im Spaltringbereich die Hämolyse von Blut nicht verhindern kann.

Die DE 196 25 300 A1 zeigt eine Blutpumpe, bei welcher der Rotor über eine zentrale Kugel gelagert ist, so dass sich Probleme der Spaltringabdichtung aufgrund dieser andersartigen Konstruktion nicht ergeben.

Die US 4,973,064 sowie die US 4,486,026 zeigen spezielle Vorrichtungen zur Magnetflüssigkeitsabdichtung, allerdings ohne auf Spezifika der hier relevanten Blutpumpen einzugehen.

Schließlich zeigt die US 4,908,012 eine relativ großbauende Blutpumpe mit einem Stator außerhalb eines gattungsgemäßen Rohres, wobei eine Spaltringabdichtung vorgesehen ist, welche durch Salzflüssigkeit fortlaufend gespült wird und sich auf diese Weise ein hydrodynamisches Lager ergibt, welches allerdings zwangsläufig nachfüllbar ist und somit bei der Nachfüllbarkeit zusätzliche Infektionsrisiken bietet.

Der Erfindung liegt die Aufgabe zugrunde, eine kleinbauende Vorrichtung zur axialen Förderung von fluiden Medien anzubieten, die eine Scherung und Verwirbelung des Fluides weitestgehend vermindert.

Die Aufgabe wird erfindungsgemaß durch eine Vorrichtung mit den Merkmalen des Anspruches 1 gelöst.

Bevorzugte und vorteilhafte Ausgestaltungen der Erfindung sind in den Unteransprüchen angegeben.

Die Erfindung bezieht sich auf einen rohrförmigen fluidführenden Hohlkörper mit einem Motor, der z. B. als Elektromotor ausgebildet sein kann, einem Laufrad mit Beschaufelung und einer Halterung für den Motor, die als Leitrad ausgebildet sein kann. Das Laufrad ist mit dem Motor kraft- und formschlüssig axial verbunden.

Der sich zwischen dem Laufrad und dem Elektromotor ausbildende Nabenspalt ist erfindungsgemäß mittels einer Magnetflüssigkeitsdichtung zum Motor hin abgedichtet. Durch die Anordnung einer Magnetflüssigkeitsdichtung im Nabenspalt kann das sonst übliche Durchströmen des Spaltes verhindert werden, so dass damit normalerweise verbundene Scherungen und Wirbelbildungen weitestgehend unterbunden werden. Kombinationen mit weiteren an sich bekannten Dichtungen sind möglich.

Weiterhin vermeidet die erfindungsgemäße Lösung eine Lagerung des Laufrades in den durchströmten Bereichen. Die Lagerung des Laufrades erfolgt ausschließlich auf der Motorwelle, so dass im Zusammenwirken mit der Magnetflüssigkeitsdichtung ein Kontakt der Lagerung mit dem durchströmenden Fluid nicht möglich ist.

Ein weiterer Vorteil der erfindungsgemäßen Losung ergibt sich daraus, dass praktisch keine Totwassergebiete im Bereich des Laufrades und des Leitrades vorhanden sind. Mögliche vorhandene Totwassergebiete im Nabenspalt zwischen Laufrad und Elektromotor sind durch die Anordnung der Magnetflüssigkeitsdichtung in die äußeren Bereiche des Nabenspaltes minimierbar.

Die erfindungsgemäß im Nabenspalt angeordnete Magnetflüssigkeitsdichtung zeigt keinen Abrieb und ist verschleiß- und reibungsarm. Magnetflüssigkeiten sind stabile Dispersionen mit superparamagnetischen Eigenschaften. Die Dispersionen bestehen im allgemeinen aus der magnetischen Komponente, aus amphiphilen Zusätzen und einer Trägerflüssigkeit. Als magnetische Komponente werden ferri- oder ferromagnetische Teilchen verwendet, deren Teilchengröße zwischen 3 und 50 nm liegt. Die Teilchen erhalten durch die sogenannten amphiphilen Zusätze entweder hydrophile oder hydrophobe Eigenschaften und können dadurch homogen entweder in wässrigen oder organischen Trägerflüssigkeiten feinverteilt werden. Als Trägerflüssigkeit kann demzufolge eine Flüssigkeit gewählt werden, die je nach zu förderndem Fluid keine Wechselwirkungsbereitschaft zeigt. Die Zusammensetzung der Magnetflüssigkeit richtet sich nach dem zu fördernden Fluid, nach dem die erwünschte Sättigungsmagnetisierung, die Viskosität und die chemische Zusammensetzung festgelegt wird. Die Sättigungsmagnetisierung bestimmt die Wechselwirkung der Magnetflüssigkeit und Magnetfeld. Je stärker die Magnetisierung ist, um so größere Druckunterschiede kann die Magnetflüssigkeitsdichtung bei sonst gleicher Magnetanordnung aushalten.

Vorteilhafterweise konnen die Magnetflüssigkeitsdichtungen mit unterschiedlichen Trägerflüssigkeiten ausgestattet werden. Das Spektrum der einsetzbaren Flüssigkeiten reicht von Wasser oder mit Wasser mischbaren Flüssigkeiten bis zu ölartigen, in Wasser unlösbaren Flüssigkeiten. Der Charakter der Trägerflüssigkeit kann dadurch dem Charakter des durch die Rohrleitungen zu transportierendem fluiden Medium angepasst werden. Werden beispielsweise wässrige Fluide gefördert, ist es zweckmäßig, eine Magnetflüssigkeit auf Ölbasis als Dichtungsmittel einzusetzen und umgekehrt. Der Grad der Wechselwirkungen zwischen Fluid und Magnetflüssigkeit geht hierbei mit Unterstützung der Wirkung des magnetischen Feldes gegen Null, was insbesondere beim Fördern und Transportieren von biologischen und sonstigen empfindlichen Fluiden wie z. B. von Blut von außerordentlichem Vorteil ist.

Die Möglichkeit, Magnetflüssigkeit auf Basis perfluorierten Polyethern einzusetzen, erlaubt es sogar, Öl-in-Wasser- bzw. Wasser-in-Öl-Emulsionen, die hydrophile und hydrophobe Eigenschaften aufweisen und damit mit einer Wechselwirkung treten könnten, mit der erfindungsgemäßen Vorrichtung zu fördern.

Diese Dichtung hat sich als außerordentlich reibungsarm erwiesen, so dass der Energieaufwand zur Erzeugung einer axialen Rotation stark minimiert werden kann und eine unzulässige Erwärmung des zu fördernden Mediums nicht auftritt.

In einer Weiterbildung der Erfindung ist ein Elektromotor in einem Leitrad integriert. Die Stromzuführung erfolgt z. B. über Halterungen des Leitrades an der Innenwandung des rohrförmigen fluidführenden Hohlkörpers.

Die Erfindung wird nachfolgend anhand einer Zeichnung mit Bezugnahme auf die Figuren näher erläutert.

Es zeigen:
- Fig. 1: eine Blutpumpe mit im Leitrad integriertem Elektromotor,
- Fig. 2 a bis Fig. 21: verschiedene schematische Ausgestaltungen der Dichtung im Nabenspaltenbereich.

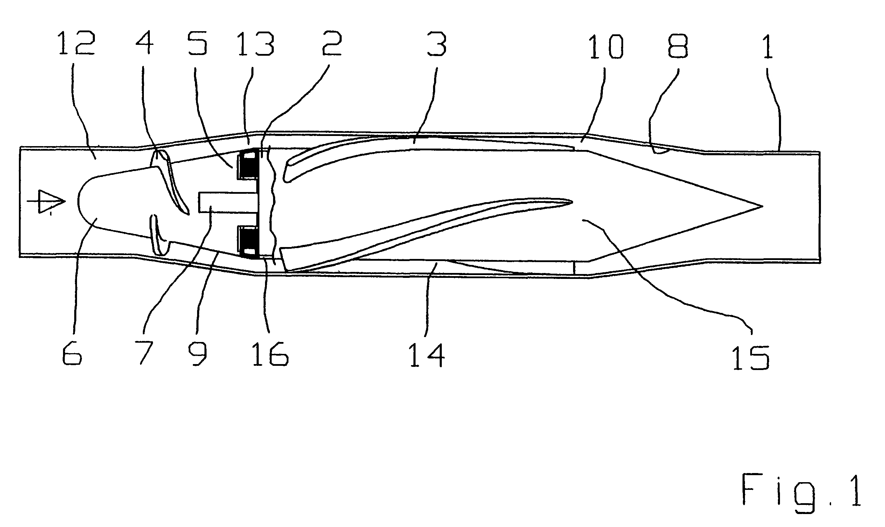

Fig. 1 zeigt schematisch eine axiale Blutpumpe. In einem rohrförmigen Hohlkörper 1 ist ein Laufrad 14 ist ein Elektromotor 2 integriert. Eine Motorwelle 7 des Elektromotors 2 ist mit dem Laufrad 9 verbunden. Zwischen Laufrad 9 und Elektromotor 2 ist eine berührungsfreie Zone, der Nabenspalt 13, vorgesehen. Der Nabenspalt 13 wird mittels einer Dichtung 5 zum durchströmenden fluiden Medium abgedichtet. Das eigentliche Dichtmedium ist eine Magnetflüssigkeit 31, die mittels einer Magnetanordnung 30 im Nabenspalt 13 fixiert wird. Der Elektromotor 2 treibt über die Motorwelle 7 das Laufrad 9 an. Die Stromversorgung des Elektromotors 2 erfolgt über hier nicht dargestellte Leitungen durch die Halterungen, die hier als Leitradschaufeln 3 fungieren, an einer Innenrohrwand 8. Im Bereich des Leitrades 14 ist der rohrförmige Hohlkörper 1 mit erweitertem Durchmesser ausgeführt. Beim Fördervorgang befördern Laufradschaufeln 4 das Blut an einer Nabe 6 vorbei in den Rotationsspalt 12, der im Bereich des Leitrades 14 in den Durchströmungsbereich übergeht. Das mittels der Laufradschaufeln 4 in Rotation versetzte Blut wird im Bereich der Leitradschaufeln 3 in axiale Richtung umgelenkt und strömt an einer Leitradnabe 15 vorbei in den rohrförmigen Hohlkörper 1.

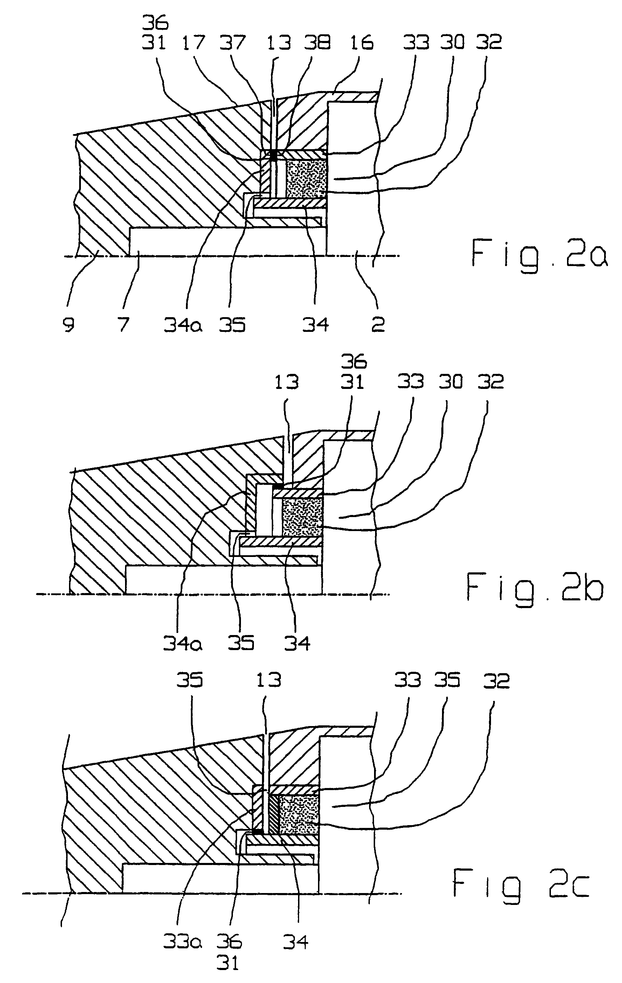

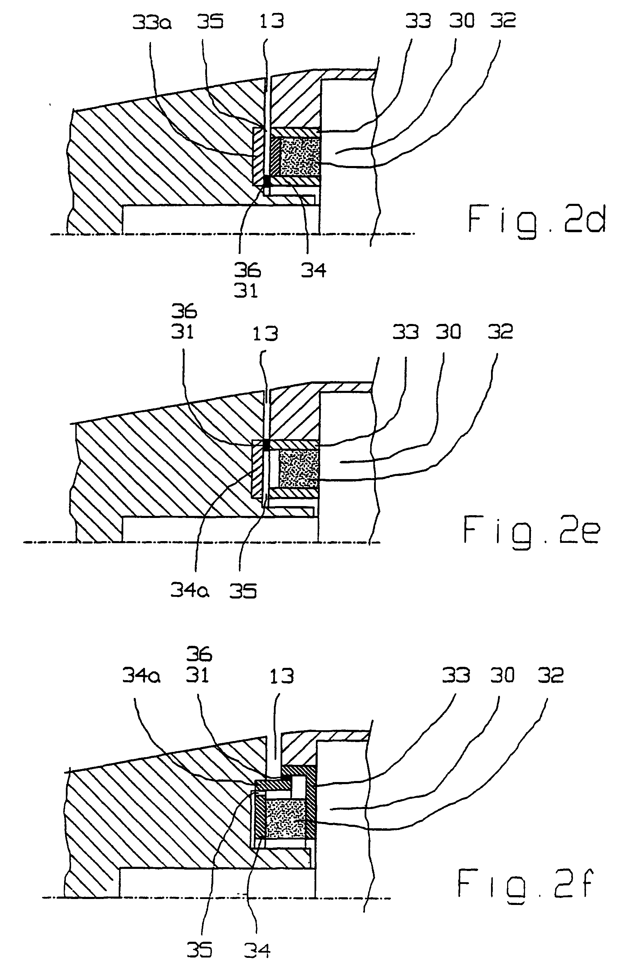

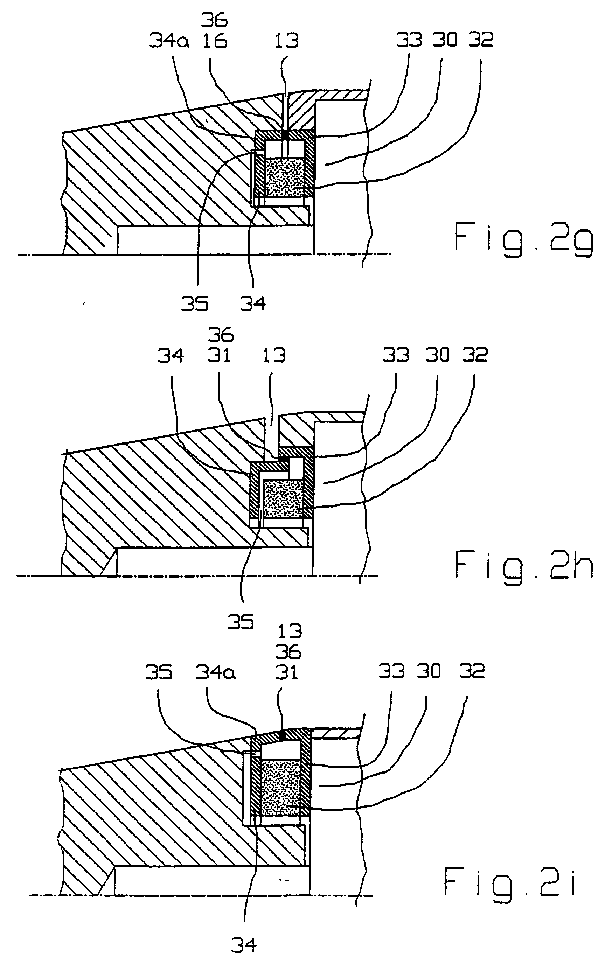

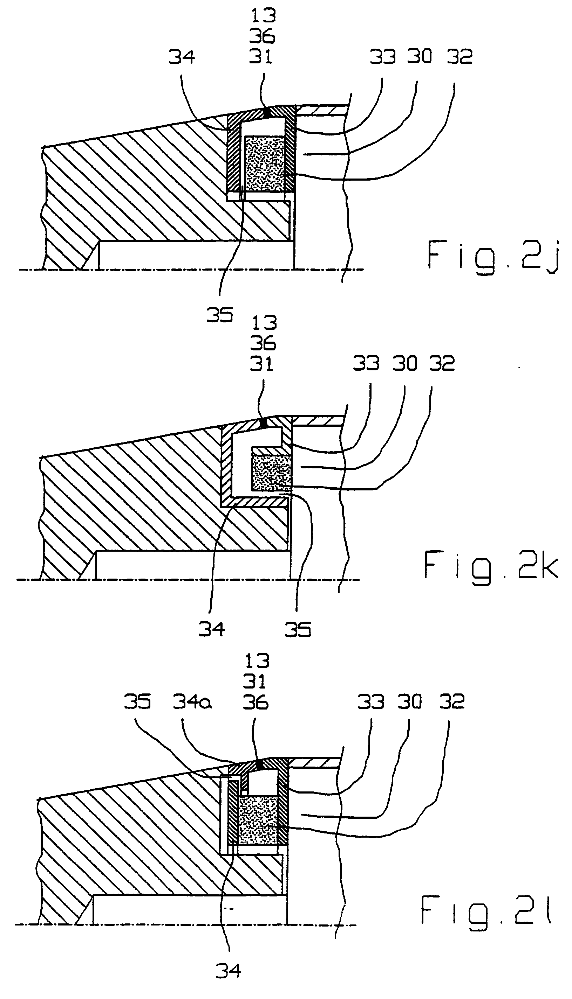

Fig. 2a bis Fig. 21 zeigen verschieden ausgeführte Dichtungen 5. Die Dichtung 5 ist sowohl in einem Laufradkopf 17 und einem Leitradkopf 16 angeordnet. Laufradkopf 17 und Leitradkopf 16 sind durch den Nabenspalt 13 voneinander getrennt. Im wesentlichen besteht die Dichtung 5 aus einer Magnetanordnung 30 mit Polschuhen 33 und 34, wobei ein Polschuh immer abgeteilte Bereiche 33a oder 34a unter Bildung eines Nebenspaltes 35 aufweist, und einer Magnetflüssigkeit 31, die in einem durch die Polschuhe 33 bzw. 33a und 34 bzw. 34a gebildeten Ringspalt angeordnet ist. Der Nebenspalt 35 ist von seiner Größe so ausgebildet, dass eine Übertragung des Magnetfeldes vom Polschuh 33 bzw. 34 auf einen Polschuh 33a bzw. 34a möglich ist und die freie Rotation des Laufrades 9 nicht behindert wird.

Die Abdichtung des Ringspaltes 36 mit der Magnetflüssigkeit 31 erfolgt in den unterschiedlichen Darstellungen radial oder axial. Entsprechend ausgebildet ist auch der Nabenspalt 35.

### Bezugszeichenliste

- 1: Hohlkörper
- 2: Elektomotor
- 3: Leitradschaufel
- 4: Laufradschaufel
- 5: Dichtung
- 6: Laufradnabe
- 7: Motorwelle
- 8: Innenrohrwand
- 9: Laufrad
- 10: Durchströmungsbereich
- 11: Stromzuführung
- 12: Rotationsspalt
- 13: Nabenspalt
- 14: Leitrad
- 15: Leitradnabe
- 16: Leitradkopf
- 17: Laufradkopf

- 30: Magnetanordnung
- 31: Magnetflüssigkeit
- 32: Magnet
- 33: Polschuh
- 33a 34: Polschuh
- 34a 35: Nebenspalt
- 36: Ringspalt
- 37: Stirnfläche
- 38: Stirnfläche

## Patentansprüche

1. Vorrichtung zur axialen Förderung von fluiden Medien, bestehend aus einem rohrformigen Hohlkörper (1), in dem ein Motor (2), mindestens ein in Rotation versetzbares Laufrad (9) mit Beschaufelung und mindestens eine Motorhalterung sowie Fluid-Leiteinrichtungen angeordnet sind, wobei das Laufrad (9) mit dem Motor (2) kraft- und/oder formschlüssig axial verbunden ist,
**dadurch gekennzeichnet, dass**
der sich zwischen der motorseitigen Stirnseite des Laufrades (9) und der laufradseitigen Stirnseite des Motors (2) ergebende Nabenspalt (13) mindestens eine ringförmige Dichtung (5) aufweist und die Dichtung (5) als Magnetflüssigkeitsdichtung ausgebildet ist.

2. Vorrichtung nach Anspruch 1,
**dadurch gekennzeichnet, dass**
die Motorhalterung als Leitrad (14) ausgebildet ist.

3. Vorrichtung nach Anspruch 1 oder 2,
**dadurch gekennzeichnet, dass**
der Motor (2) als Elektromotor ausgebildet ist.

4. Vorrichtung nach einem Anspruch 3,
**dadurch gekennzeichnet, dass**
der Elektromotor (2) in ein Leitrad (14) integriert ist.

5. Vorrichtung nach einem der Ansprüche 2 bis 4,
**dadurch gekennzeichnet, dass**
das Leitrad (14) an einer Innenrohrwand (8) des rohrförmigen Hohlkörpers (1) gehaltert ist.

6. Vorrichtung nach Anspruch 5,
**dadurch gekennzeichnet, dass**
die Halterung des Leitrades (14) eine Stromzufuhrung (11) aufweist.

7. Vorrichtung nach Anspruch 5 oder 6,
**dadurch gekennzeichnet, dass**
die Halterungen des Leitrades (14) als Leitradschaufeln (3) ausgebildet sind.

8. Vorrichtung nach einem der Ansprüche 1 bis 7,
**dadurch gekennzeichnet, dass**
die Dichtung (5) aus einer Magnetanordnung (30) und einer von ihr fixierten Magnetflüssigkeit (31) besteht.

9. Vorrichtung nach Anspruch 8,
**dadurch gekennzeichnet, dass**
die Magnetanordnung (30) aus einem Magnet (32) und zwei einen Ringspalt (36) bildenden, eine unterschiedliche magnetische Polung aufweisenden Polschuhen (33, 34) besteht, zwischen deren Stirnflächen (37, 38) die Magnetflüssigkeit (31) angeordnet ist, wobei Magnet (32), Polschuh (33) und/oder Polschuh (34) am Laufrad (9) und/oder am Elektromotor (2) fixiert sind.

10. Vorrichtung nach Anspruch 9,
**dadurch gekennzeichnet, dass**
ein Polschuh (33 oder 34) zweiteilig, durch einen Nebenspalt (35) getrennt, ausgebildet ist.

11. Vorrichtung nach einem der Ansprüche 9 oder 10,
**dadurch gekennzeichnet, dass**
die den Ringspalt (36) begrenzenden Stirnflächen (37, 38), zwischen denen die Magnetflüssigkeit (31) angeordnet ist, zueinander spiegelsymmetrisch ausgebildet sind.

12. Vorrichtung nach einem der Ansprüche 9 oder 10,
**dadurch gekennzeichnet, dass**
die den Ringspalt (36) begrenzenden Stirnflächen (37, 38) zueinander nicht symmetrisch ausgebildet sind.

13. Vorrichtung nach einem der Ansprüche 9 bis 12,
**dadurch gekennzeichnet, dass**
die Stirnflächen (37, 38) umlaufend gefertigte Vertiefungen und/oder Erhöhungen aufweisen.

14. Vorrichtung nach einem der Ansprüche 9 bis 12,
**dadurch gekennzeichnet, dass**
die Stirnflächen (37, 38) eine plane Ausbildung aufweisen.

15. Vorrichtung nach einem der Ansprüche 9 bis 14,
**dadurch gekennzeichnet, dass**
die Stirnflächen (37, 38) parallel zueinander angeordnet sind.

16. Vorrichtung nach einem der Ansprüche 9 bis 14,
**dadurch gekennzeichnet, dass**
die Stirnflächen (37, 38) nicht parallel angeordnet sind.

17. Vorrichtung nach einem der Ansprüche 9 bis 16,
**dadurch gekennzeichnet, dass**
die Stirnflächen (37, 38) bezogen auf die Ringspaltachse rechtwinklig angeordnet sind.

18. Vorrichtung nach einem der Ansprüche 9 bis 16,
**dadurch gekennzeichnet, dass**
die Stirnflächen (37, 38) bezogen auf die Ringspaltachse spitz- und/oder stumpfwinklig angeordnet sind.

19. Vorrichtung nach einem der Ansprüche 9 bis 16,
**dadurch gekennzeichnet, dass**
die Stirnflächen (37, 38) rundum konkav und/oder konvex gewölbt sind.

20. Vorrichtung nach einem der Ansprüche 8 bis 19,
**dadurch gekennzeichnet, dass**
die Magnete (32) der Magnetanordnung (30) als Permanent- oder Elektromagnete ausgebildet sind.

21. Vorrichtung nach einem der Ansprüche 8 bis 20,
**dadurch gekennzeichnet, dass**
die Magnetflüssigkeitsdichtung mit mindestens einer an sich bekannten Dichtung kombiniert ist.

## Claims

1. Device for axially delivering fluidic media consisting of a tubular hollow body (1), in which a motor (2), at least one impeller (9) with blading which can be rotated and at least one motor mounting as well as fluid stator means are arranged, wherein the impeller (9) is connected axially force-fittingly and/or form-fittingly to the motor (2),
**characterized in that**
the hub gap (13) resulting between the motor-side front face of the impeller (9) and the impeller-side front fact of the motor (2) is provided with at least one annular seal (5) and the seal (5) is formed as a magnetic particle seal.

2. Device according to claim 1,
**characterized in that**
the motor mounting is formed as a stator (14).

3. Devise according to claim 1 or 2,
**characterized in that**
the motor (2) is formed as an electric motor.

4. Device according to claim 3,
**characterized in that**
the electric motor (2) is integrated in a stator (14).

5. Device according to one of claims 2 to 4,
**characterized in that**
the stator (14) is mounted on an inner pipe wall (8) of the tubular hollow body (1).

6. Device according to claim 5,
**characterized in that**
the mounting of the stator (14) has a power supply (11).

7. Device according to claim 5 or 6,
**characterized in that**
the mountings of the stator (14) are formed as stator blades (3).

8. Device according to one of claims 1 to 7,
**characterized in that**
the seal (5) consists of a magnet arrangement (30) and of a magnetic liquid (31) held by it.

9. Device according to claim 8,
**characterized in that**
the magnet arrangement (30) consists of a magnet (32) and two pole shoes (33, 34) forming an annular gap (36) and having a different magnetic charge, between which end faces (37, 38) a magnetic liquid (31) is arranged, wherein the magnet (32), the pole shoe (33) and/or the pole shoe (34) are fixed on the impeller (9) and/or on the electric motor (2).

10. Device according to claim 9,
**characterized in that**
a pole shoe (33 or 34) is formed in two pieces, separated by a secondary gap (35).

11. Device according to one of claims 9 or 10,
**characterized in that**
the end faces (37, 38) limiting the annular gap (36), between which the magnetic liquid (31) is arranged, are formed mirror symmetrically to each other.

12. Device according to one of claims 9 or 10,
**characterized in that**
the end faces (37, 38) delimiting the annular gap (36) are not formed symmetrically to each other.

13. Device according to one of claims 9 to 12,
**characterized in that**
the end faces (37, 38) have recesses and/or projections formed circumferentially.

14. Device according to one of claims 9 to 12,
**characterized in that**
the end faces (37, 38) are formed planar.

15. Device according to one of claims 9 to 14,
**characterized in that**
the end faces (37, 38) are arranged parallel to each other.

16. Device according to one of claims 9 to 14,
**characterized in that**
the end faces (37, 38) are not arranged parallel to each other.

17. Device according to one of claims 9 to 16,
**characterized in that**
the end faces (37, 38) are arranged at a right angle to the annular gap axis.

18. Device according to one of claims 9 to 16,
**characterized in that**
the end faces (37, 38) are arranged acute-angled and/or obtus-angled to the annular gap axis.

19. Device according to one of claims 9 to 16,
**characterized in that**
the end faces (37, 38) circumferentially ar formed concave and/or convex.

20. Device according to one of claims 8 to 19,
**characterized in that**
the magnets (32) of the magnet arrangement (30) are formed as permanent or electromagnets.

21. Device according to one of claims 8 to 20,
**characterized in that**
the magnetic particle seal is combined with at least one seal of the state of the art.

## Revendications

1. Dispositif pour l'alimentation axiale de substances fluides, comportant un corps creux (1) tubulaire, dans lequel sont agencés un moteur (2), au moins un rotor (9) à aubage, apte à être entraîné en rotation, et au moins un support de moteur, ainsi que des dispositifs de guidage du fluide, le rotor (9) étant relié axialement au moteur (2) par adhérence et/ou par emboîtement, **caractérisé en ce que** la fente du moyeu (13), se formant entre la face frontale côté moteur du rotor (9) et la face frontale côté rotor du moteur (2), contient au moins une garniture d'étanchéité (5) annulaire et la garniture d'étanchéité (5) est conçue sous forme de garniture d'étanchéité à liquide magnétique.

2. Dispositif selon la revendication 1, **caractérisé en ce que** le support de moteur est conçu sous forme de roue de guidage (14).

3. Dispositif selon la revendication 1 ou 2, **caractérisé en ce que** le moteur (2) est conçu sous forme de moteur électrique.

4. Dispositif selon la revendication 3, **caractérisé en ce que** le moteur électrique (2) est intégré dans une roue de guidage (14).

5. Dispositif selon l'une quelconque des revendications 2 à 4, **caractérisé en ce que** la roue de guidage (14) est maintenue sur une paroi tubulaire intérieure (8) du corps creux (1) tubulaire.

6. Dispositif selon la revendication 5, **caractérisé en ce que** le système de maintien de la roue de guidage (14) comporte une alimentation en courant (11).

7. Dispositif selon la revendication 5 ou 6, **caractérisé en ce que** les systèmes de maintien de la roue de guidage (14) sont conçus sous forme d'aubes (3) de la roue de guidage.

8. Dispositif selon l'une quelconque des revendications 1 à 7, **caractérisé en ce que** la garniture d'étanchéité (5) est formée par un système magnétique (30) et un liquide magnétique (31) bloqué par celui-ci.

9. Dispositif selon la revendication 8, **caractérisé en ce que** le système magnétique (30) est formé par un aimant (32) et deux épanouissements polaires (33, 34) à polarité magnétique différente, formant une fente annulaire (36) et entre les faces frontales (37, 38) desquels est disposé le liquide magnétique (31), l'aimant (32), l'épanouissement polaire (33) et/ou l'épanouissement polaire (34) étant fixés sur le rotor (9) et/ou sur le moteur électrique (2).

10. Dispositif selon la revendication 9, **caractérisé en ce qu'**un épanouissement polaire (33 ou 34) est réalisé en deux parties séparées par une fente secondaire (35).

11. Dispositif selon la revendication 9 ou 10, **caractérisé en ce que** les faces frontales (37, 38) délimitant la fente annulaire (36) et entre lesquelles est disposé le liquide magnétique (31), sont réalisées symétriquement l'une de l'autre.

12. Dispositif selon la revendication 9 ou 10, **caractérisé en ce que** les faces frontales (37, 38) délimitant la fente annulaire (36) ne sont pas réalisées symétriquement l'une de l'autre.

13. Dispositif selon l'une des revendications 9 à 12, **caractérisé en ce que** les faces frontales (37, 38) comportent des concavités et/ou convexités réalisées sur le pourtour.

14. Dispositif selon l'une des revendications 9 à 12, **caractérisé en ce que** les faces frontales (37, 38) sont planes.

15. Dispositif selon l'une des revendications 9 à 14, **caractérisé en ce que** les faces frontales (37, 38) sont agencées parallèlement l'une à l'autre.

16. Dispositif selon l'une des revendications 9 à 14, **caractérisé en ce que** les faces frontales (37, 38) ne sont pas agencées parallèlement l'une à l'autre.

17. Dispositif selon l'une des revendications 9 à 16, **caractérisé en ce que** les faces frontales (37, 38) sont agencées à angle droit par rapport à l'axe de la fente annulaire.

18. Dispositif selon l'une des revendications 9 à 16, **caractérisé en ce que** les faces frontales (37, 38) sont agencées en angle aigu et/ou en angle obtus par rapport à l'axe de la fente annulaire.

19. Dispositif selon l'une quelconque des revendications 9 à 16, **caractérisé en ce que** les faces frontales (37, 38) sont courbes sur le pourtour de manière concave et/ou convexe.

20. Dispositif selon l'une quelconque des revendications 8 à 19, **caractérisé en ce que** les aimants (32) du système magnétique (30) sont conçus sous forme d'aimants permanents ou d'électroaimants.

21. Dispositif selon l'une quelconque des revendications 8 à 20, **caractérisé en ce que** la garniture d'étanchéité à liquide magnétique est combinée à au moins une garniture d'étanchéité connue en soi.
